# EUROPÄISCHE PATENTSCHRIFT

(11) **EP 1 119 393 B1**
(45) Veröffentlichungstag und Bekanntmachung des Hinweises auf die Patenterteilung: **23.04.2003**
(21) Anmeldenummer: 99952746.8
(22) Anmeldetag: 08.10.1999
(51) Int. Cl.: A61N 2/02

(54) **ELEKTROMAGNETISCHE STIMULIERUNG VON KNORPELGEWEBE UNTER VERWENDUNG VON FGF**
ELECTROMAGNETICALLY STIMULATING CARTILAGE TISSUE USING FGF
STIMULATION ELECTROMAGNETIQUE DE TISSUS CARTILAGINEUX UTILISANT FGF

(30) Priorität: 09.10.1998 DE 19846685
(43) Veröffentlichungstag der Anmeldung: 01.08.2001
(73) Patentinhaber: Markoll, Richard, 81925 Munchen (DE)
(72) Erfinder: Markoll, Richard, 81925 Munchen (DE)
(74) Vertreter: HOFFMANN - EITLE
(86) Internationale Anmeldenummer: IB9901807
(87) Internationale Veröffentlichungsnummer: WO00021611

(56) Entgegenhaltungen:
- WO-A-85/00293
- US-A- 5 067 940
- US-A- 5 330 410
- US-A- 5 620 463

## Beschreibung

Die Erfindung betrifft die Verwendung von FGF zur Herstellung eines Medikaments.

Insbesondere betrifft sie die Verwendung von FGF (Fibroblast Growth Factor) zusammen mit pulsierenden elektromagnetischen Signalen zur Stimulierung von Knorpel und Knorpelbildung.

Es ist bekannt, daß gesunder Gelenkknorpel unter physiologischen Bedingungen belastet wird und daß dies zu Knorpelverschleiß mit Zerstörung der Knorpeloberflächen führen kann. Auch Inaktivität und durch Entzündungen verursachte Veränderungen der Synovia können zur Knorpelzerstörung führen.

Knorpel ist ein Stützgewebe, das aus wasserreichen Knorpelzellen (Chondrozyten) und Interzellularsubstanz besteht. Die extrazelluläre Matrix des Knorpels besteht zu 60 bis 80 % aus Wasser. Die Interzellularsubstanz setzt sich aus Grundsubstanz (Proteoglykanen und Glukoproteinen) sowie Fasern (Kollagenen) zusammen. Proteoglykane, Glukoproteine und Kollagene werden von Chondrozyten hergestellt. Die Kollagene bilden ein Geflecht, worin Proteoglykane und Glukoproteine eingelagert und fixiert sind.

Es gibt viele verschiedene Arten von Kollagenen. Kollagenmoleküle des Typs I, II, III, V und VI haben ähnliche Molekularstrukturen. Kollagenmoleküle des Typs IX, XII und XIV enthalten mehrere Tripelhelix-Domänen, die durch nichtkollagene Domänen unterbrochen sind. Innerhalb der verschiedenen Typen von Kollagenmolekülen lassen sich auch signifikante Unterschiede feststellen. Kollagenmoleküle des Typs V haben einen niedrigen Alaningehalt, aber einen hohen Gehalt an basischen Aminosäuren. Außerdem schließen sie sich zu Tetrameren zusammen. Der Typ VIII ist eine starke Protease. Der Typ IX ist auf der Oberfläche von Kollagen Typ II zu finden. Kollagenmoleküle des Typs XI sind kurze Tripelhelices mit sehr langen globulären Ausdehnungen. Kollagenmoleküle des Typs VII haben einen N-terminalen Domänenbereich mit drei Fingern, ähnlich den Kollagentypen XII und XIV.

Die Proteoglykane bilden hydratisierte Gele, wodurch Nährstoffe die Chondrozyten durch Diffusion erreichen. Proteoglykane sind Makromoleküle, die aus einem Proteinkern bestehen, an den mehrere Glykosaminglycanketten gebunden sind. Bestandteile der Glykosaminglycane sind Disaccharide mit Sulfat- und Carboxylgruppen mit negativen Ladungen, die die Ladungsdichte der extrazellulären Knorpelmatrix bestimmen.

Diese negativen Ladungen werden durch Natriumionen aus der extrazellulären Flüssigkeit neutralisiert. Aus diesem Grunde ist eine beträchtliche Menge Natrium im Knorpel enthalten, wodurch ein hoher osmotischer Druck entsteht, der Wasser anzieht, weshalb der Knorpel zu 80 % aus Wasser besteht.

Bei Belastung wird der Knorpel zusammengedrückt, das Wasser wird verdrängt und nimmt dabei die mobilen Natriumionen mit sich, während die negativ geladenen Carboxyl- und Sulfationen des Proteoglykans zurückbleiben. Dieser Vorgang erzeugt elektrischen Strom, da nicht-neutralisierte negative Ladungen im Knorpel vorliegen. Nach dem Donnan-Effekt bestimmen negative Ladungen die Konzentration der Gegenionen. Die negativen Ladungen und der Elektronenfluß erzeugen eine elektrische Ladung. Bei Entlastung werden die Natriumionen und das Wasser wieder angezogen und der Knorpel dehnt sich erneut bis auf sein ursprüngliches Volumen aus.

Durch diesen Mechanismus werden die Chondrozyten zur Synthese zusätzlicher Matrix angeregt. Die Biosyntheseleistung der Chondrozyten hängt von der extrazellulären Osmolarität ab, weshalb anzunehmen ist, daß mechanische und physiochemische Bedingungen eine wichtige Voraussetzung für die Übermittlung von Signalen in Verbindung mit der applizierten Belastung ist.

Die Strömungspotentiale des Knorpels können durch Verminderung des Proteoglykangehalts, zum Beispiel durch enzymatischen Abbau der Chondrozyten, verändert werden. Die Veränderungen der extrazellulären Matrix müssen also auch zur Veränderung der elektrischen Phänomene führen, so daß der Chondrozyt veränderte Signale erhält, die seine Syntheseleistung beeinflussen.

Bei Osteoarthritis tritt eine Abnutzung des Knorpels auf, so daß die Matrix verloren geht. Der Knorpel kann seine normale Funktion durch Verlust der Matrixkomponenten nicht mehr ausüben, da die vorhandene Menge Proteoglykan geringer ist. Außerdem ist im Labor festgestellt worden, daß beanspruchter Knorpel und andere benachbarte Gewebe des Gelenkes sich verdicken, und demnach weniger Strom fließen kann.

Bekannt ist die sogenannte Magnetfeldtherapie (Vgl. US-A-5 330 410), die bei nicht heilenden Knochenbrüchen angewendet wird. Bei der Magnetfeldtherapie wird sinusförmiger, kontinuierlicher Wechselstrom mit einer Frequenz von circa 44 bis 77 Hz und einer Feldstärke von 2 G verwendet.

Von FGF ist bekannt, daß er ein Wachstumsstimulans für Fibroblasten ist. FGF ist in basischen FGF (bFGF) und sauren FGF (aFGF) zu unterteilen. bFGF ist in Knorpel und Knochen vorhanden und bei der Entwicklung und dem Wachstum von Knorpeln beteiligt. Über verschiedene Formen von bFGF wurde im Kern und Cytoplasma und in der extrazellulären Matrix von Zellen berichtet. Die Verwendung von bFGF induziert Chondrozytenvermehrung und extrazelluläre Matrixsynthese (siehe Cuevas, P., Burgos, J. und Baird, A., Basic fibroblast growth factor (FGF) promotes cartilage repair in vivo, Biochem. Biophys. Res. Commun., 31, 611, 1988). Außerdem wurde festgestellt, daß die Injektion von aFGF (1 µg/d) an der Stelle des Knochenbruchs die Bildung von Knorpel und Knochen steigert (siehe Jingushi, S., Heydemann, A., Kana, S.K., Macey, L.R. und Bolander, M.E., Acidic fibroblast growth factor (aFGF) injection stimulates cartilage enlargement and inhibits cartilage gene expression in rat fracture healing, J. Orthop. Res., 8, 364, 1990).

### Beschreibung der Abbildungen

Figur 1:
Bild 1: Ladungsausgleich zwischen Wasserstoffprotonen und negativen Ladungsträgern der extrazellulären Knorpelmatrix.
Bild 2: Entstehung eines Spannungspotentiales im Gelenk bei Belastung durch "Auspressen" von Flüssigkeit aus dem Knorpelgewebe mit Verschiebung der Wasserstoffprotonen.
Bild 3: Erzeugung sehr vieler, unterschiedlich strömender Potentiale im Gelenk über eine erzwungene Wanderung der Wasserstoffprotonen in der EZM durch wechselnde Rechteckimpulse als Stimulierungsreiz für die Zellen des Bindegewebes, in erster Linie der Chondrozyten.

Figur 2:
Bild 1: Diagramm einer Krause-Lechner Spule, die mit wechselstromgerichtetem Magnetfeld arbeitet, wodurch eine Sinuskurve erzeugt wird. Diese Form der Energieübertragung ist nicht physiologisch.
Bild 2: Diagramm, das die Anwendung gepulster elektromagnetischer Felder zeigt. Ein gleichstromgerichtetes, sich ständig wiederholendes Signal wird verwendet. Dieses wird mit einer bestimmten Intensität und einer bestimmten Frequenz übertragen. Der Impuls ist für die Dauer der Entwicklung auf das Gelenk konstant.
Bild 3: Diagramm mit wechselnden Rechteckimpulsen als Stimulierung, die für die Dauer der Behandlung alternierend übertragen werden. Die Intensität der Recheckimpulse liegt zum Größteil im Bereich zwischen 0,5 und 1,5 Millitessla. Die Frequenz bewegt sich in einem Bereich zwischen 10 und 30 Hertz. Diese Form der Stimulierung liegt sowohl in einem relativ niedrig frequenten, als auch, was die Feldstärke betrifft, in einem niedrigen energetischen Bereich.

### Detaillierte Beschreibung der Erfindung

Aufgabe dieser Erfindung ist die Regeneration von Knorpel bei Knorpelschäden. Insbesondere soll dabei die Bildung von Kollagen-Typen stimuliert werden, die im gesunden Knorpelgewebe vorhanden sind.

Diese Aufgabe wird dadurch gelöst, dass FGF zur Stimulierung von Knorpelgewebe bei solchen Patienten verwendet wird, die gleichzeitig elektromagnetischen Signalen ausgesetzt sind.

Gemäß der Erfindung wird die Aufgabe durch den Gegenstand der Ansprüche gelöst.

Es wurde festgestellt, daß durch die Verwendung von elektromagnetischen Signalen, erzeugt durch pulsierenden, impulsmodulierten, quasi-rechteckigen Gleichstrom, wobei die Frequenz 1-30 Hz, bevorzugt 10-12 Hz und die Feldstärke 10-20 G, bevorzugt 12-14 G, besonders bevorzugt 12,5 G beträgt, Knorpelgewebe besonders gut stimuliert wird. Besonders vorteilhaft ist es dabei, daß die Modulationsform quasi rechteckig ist. Bei den erfindungsgemäß verwendeten elektromagnetischen Signalen handelt es sich um pulsierende Signale. Es werden dabei schwache elektromagnetische Felder in den Körper gesandt. Sie bewirken den Aufbau eines elektrischen Feldes. Durch die erzeugten elektromagnetischen Signale wird der geschädigte Knorpel einem elektromagnetischen Feld ausgesetzt, das dem entspricht, wie es im gesunden Knorpel vorgefunden wird. Erfolgt dies über einen bestimmten Zeitraum, so wird auf diese Weise die Stimulierung von Knorpelgewebe nach dessen Schädigung sowie das Wachstum von Chondrozyten bewirkt.

Diese Form der Stimulierung bewirkt also, daß der Chondrozyt, der durch pathologische Veränderung der extrazellulären Matrix keine physiologischen Signale mehr empfängt, wieder Signale empfängt und dadurch der Metabolismus von Bindegeweben, hyalinem Knorpel, Faserknorpeln, Bändern, Sehnen und Gelenkkapseln normalisiert werden kann. Dies wird auch durch die klinische Beobachtung einer deutlichen Schmerzreduktion bei Anwendung dieser Therapie bestätigt.

Die Verwendung der erfindungsgemäßen Signale erzeugt in Ruhe ohne Belastung sehr viele unterschiedliche strömende Potentiale im Gelenk. Erreicht wird dies durch ein wechselndes Muster an Recheckimpulsen, hierdurch werden Wasserstoffprotonen zu unterschiedlichen Wanderungen im Gelenkbereich gezwungen. Infolgedessen werden sehr viele multidirektionale strömende Potentiale hervorgerufen, die zu einer Aktivitätssteigerung der Chondrozyten führen.

Erfindungsgemäß ist es erforderlich, die Signale des elektromagnetischen Feldes so einzustellen, daß sie den natürlichen Verhältnissen in Knorpelgewebe nahekommen.

Bewährte Indikationen für die Anwendung von derartigen Signalen sind Erkrankungen des Bewegungsapparates, wie zum Beispiel Arthrosen, Tendinitis, Formenkreise der rheumatischen Erkrankungen, Rheuma und akute Verletzungen.

Experimentelle Untersuchungen mit elektromagnetischen Signalen mit einer Pulsdauer von 30 ms, einer Frequenz von 1,5 Hz und einem Impulsanstieg innerhalb von 230 ms an Knorpelexplantatkulturen haben gezeigt, daß eine ausgeglichene Proteoglykanzusammensetzung erhalten bleibt, ohne daß die molekulare Struktur und Funktion beeinflußt wird.

Gemäß der Erfindung ist die Verwendung von FGF zur Stimulierung von Knorpelgewebe bei Patienten, die gleichzeitig elektromagnetischen Signalen, erzeugt durch pulsierenden, impulsmodulierten Gleichstrom mit einer Frequenz von 1 bis 30 Hz und einer Feldstärke von 10 bis 20 G ausgesetzt sind.

Bei der Verwendung der erfindungsgemäßen pulsierenden Signale zur Stimulierung von Knorpelgewebe wird FGF intramuskulär, intravenös oder subkutan verabreicht.

Dabei erhalten die Patienten eine Dosis von bis zu 100 µg FGF. Bevorzugt wird die Behandlung in 9 einstündigen Abschnitten an möglichst aufeinanderfolgenden Tagen durchgeführt.

Es wurde außerdem festgestellt, daß bei Verwendung von FGF bei Patienten, die gleichzeitig elektromagnetischen Signalen mit den obigen Merkmalen ausgesetzt sind, die Bildung von Knorpelgewebe stimuliert wird, wobei insbesondere die Synthese von Kollagen II, IX, XI und XII stimuliert wird.

In Tabelle 1 sind die für die Behandlung von Knorpel verwendeten Energie-Charakteristika gezeigt, die sich als besonders wirksam erwiesen.

**Tabelle 1**

| **Parameter** | **PST** |
|---|---|
| Energieform | Gleichstrom |
| Frequenz | 1 - 30 Hz |
| Wellenform | quasi rechteckig |
| Feldstärke | 10 - 20 G |
| Impulsfrequenz | impulsmoduliert |
| Frequenzquelle | 6 Frequenzquellen |
| Implementierung | Freilaufdiode |
| Energietreiber | pulsierender Gleichstrom |
| Arbeitszyklus | > 60 % |

Außerdem wurden mehrere Enzyme und Syntheseprodukte im Knorpel bzw. in der Synovialflüssigkeit nachgewiesen. Von der Konzentration bestimmter Knorpelmarker können Rückschlüsse auf degradative und reparative Vorgänge im Gelenkknorpel gezogen werden.

Knorpelmarker wurden vor und nach Behandlung mit pulsierenden elektromagnetischen Signalen des vorgenannten Typs bestimmt. Folgende Parameter wurden in der Synovialflüssigkeit bestimmt: Die Matrixoproteasen (Kollagenasen) MMP-I, MMP-III, MMP-VIII, der MMP-Inhibitor TIMP 1, die mit der Bindegewebsproliferation assoziierten Matrixproteine Tenaszin und PIIINP, die mit der synovialen Entzündung assozierten Zytokine TNF-α, TGF-β1, IL-1 und IL-6 sowie CRP, Harnstoff und das Gesamteiweiß mit Albuminfraktion.

Außerdem kann in der Synovialflüssigkeit ein Marker, wie Harnstoff oder Albumin, bestimmt werden.

Die Messung von Tenazin liefert Ergebnisse über die Synthese und Proliferation von Knorpel, während MMP-2 über die Kollagendegradation und TIMP-1 über die Matrixsynthese informiert.

Die Veränderungen der Syntheseleistung und der Degradation werden im Gelenkknorpel nachgewiesen. Durch die Untersuchung der Gelenkflüssigkeit vor und nach der Behandlung konnte der positive Einfluß der erfindungsgemäßen elektromagnetischen Signale auf den Knorpelstoffwechsel nachgewiesen werden.

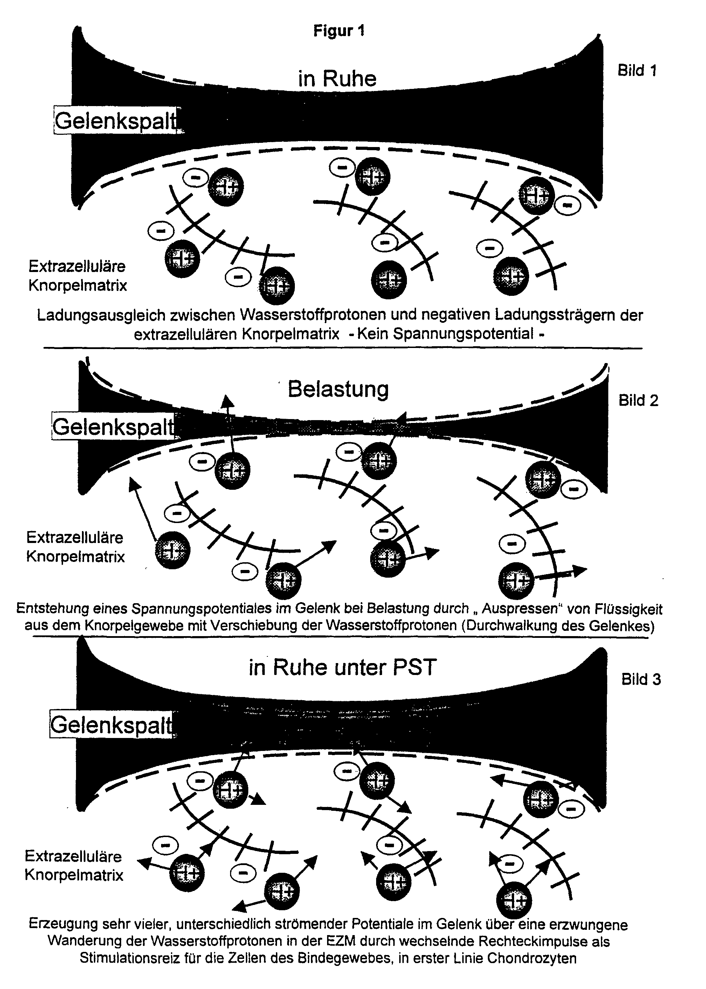

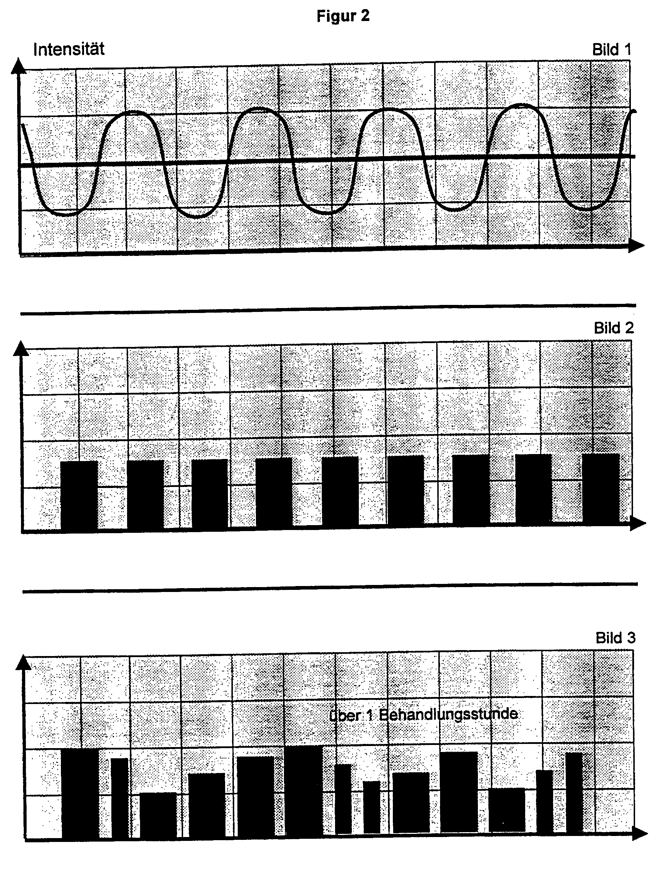

## Patentansprüche

1. Verwendung von FGF zur Herstellung eines Medikaments zur Stimulierung von Knorpelgewebe bei Patienten, die gleichzeitig elektromagnetischen Signalen, erzeugt durch pulsierenden impulsmodulierten Gleichstrom, wobei die Frequenz 1 bis 30 Hz und die Feldstärke 10 bis 20 G ist, ausgesetzt sind.

2. Verwendung nach Anspruch 1, **dadurch gekennzeichnet, daß** die Modulationsform quasi rechteckig ist.

3. Verwendung nach Anspruch 1 oder 2, **dadurch gekennzeichnet, daß** die Frequenz 10 bis 12 Hz beträgt.

4. Verwendung nach Ansprüchen 1 bis 3, **dadurch gekennzeichnet, daß** die Feldstärke 12-14 G beträgt.

5. Verwendung nach Anspruch 4, **dadurch gekennzeichnet, daß** die Feldstärke 12,5 G beträgt.

6. Verwendung nach Ansprüchen 1 bis 5, wobei die Pulszüge moduliert sind.

7. Verwendung nach Ansprüchen 1 bis 6, zur Bildung von Kollagen II, IX, XI und XII zur Stimulierung von Knorpelgewebe.

8. Verwendung nach Ansprüchen 1 bis 8, wobei FGF intramuskulär, intravenös oder subkutan verabreicht wird.

## Claims

1. Use of FGF for producing a medicament for stimulating cartilage in patients, who are exposed at the same time to electromagnetic signals produced by pulsating pulse-modulated direct current, wherein the frequency is 1 to 30 Hz and the field strength 10 to 20 G.

2. Use according to claim 1, **characterised in that** the modulation shape is quasi-rectangular.

3. Use according to claim 1 or 2, **characterised in that** the frequency is 10 to 12 Hz.

4. Use according to claims 1 to 3, **characterised in that** the field strength is 12-14 G.

5. Use according to claim 4, **characterised in that** the field strength is 12.5 G.

6. Use according to claims 1 to 5, wherein the pulse trains are modulated.

7. Use according to claims 1 to 6, for forming collagen II, IX, XI and XII for stimulating cartilage.

8. Use according to claims 1 to 8, wherein FGF is administered intramuscularly, intravenously or subcutaneously.

## Revendications

1. Utilisation de FGF (facteur de croissance des fibroblastes) pour la fabrication d'un médicament pour la stimulation des tissus cartilagineux chez des patients, qui sont exposés simultanément à des signaux électromagnétiques, produits par un courant continu pulsatoire à modulation d'impulsion, la fréquence étant de 1 à 30 Hz et l'intensité de champ étant de 10 à 20 G.

2. Utilisation selon la revendication 1, **caractérisée en ce que** la forme de modulation est quasi rectangulaire.

3. Utilisation selon l'une des revendications 1 ou 2, **caractérisée en ce que** la fréquence est de 10 à 12 Hz.

4. Utilisation selon les revendications 1 à 3, **caractérisée en ce que** l'intensité de champs est de 12 à 14 G.

5. Utilisation selon la revendication 4, **caractérisée en ce que** l'intensité de champ est de 12,5 G.

6. Utilisation selon les revendications 1 à 5, les trains d'impulsion étant modulés.

7. Utilisation selon les revendications 1 à 6, pour former du collagène II, IX, XI et XII pour la stimulation des tissus cartilagineux.

8. Utilisation selon les revendications 1 à 8, le FGF étant administré par voie intramusculaire, intraveineuse, ou sous-cutanée.
